(19)

Europäisches Patentamt

European Patent Office

Office européen des brevets

(11) **EP 4 278 966 A1**

(12) **EUROPEAN PATENT APPLICATION**

(43) Date of publication:
**22.11.2023 Bulletin 2023/47**

(21) Application number: **22173589.7**

(22) Date of filing: **16.05.2022**

(51) International Patent Classification (IPC):
**A61B 5/145** (2006.01)   **A61B 5/1473** (2006.01)
**A61B 5/1486** (2006.01)   **A61B 5/00** (2006.01)

(52) Cooperative Patent Classification (CPC):
**A61B 5/14532; A61B 5/1451; A61B 5/14735;
A61B 5/14865;** A61B 5/7242; A61B 2560/0468;
A61B 2562/0217; A61B 2562/125

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Roche Diabetes Care GmbH
68305 Mannheim (DE)**
Designated Contracting States:
**DE**
• **F. Hoffmann-La Roche AG
4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**

(72) Inventor: **PÖTSCHKE, Markus
68305 Mannheim (DE)**

(74) Representative: **Grünecker Patent- und
Rechtsanwälte
PartG mbB
Leopoldstraße 4
80802 München (DE)**

(54) **ANALYTE SENSOR MORPHOLOGY**

(57)   The invention relates to an analyte sensor for use in medical devices for measuring analyte data in an analyte carrying fluid, in particular for measuring glucose data, comprising: a first electrode (101) being a working electrode, a second electrode, a substrate (100) carrying the first electrode and the second electrode, at least one membrane (104) which at least partially covers the first electrode (101), wherein the at least one membrane (101) comprises a membrane material that is impermeable to the analyte carrying fluid and/or the analyte, wherein the at least one membrane comprises at least one opening (107), and wherein the at least one membrane (104) is configured for controlling the flux of the analyte carrying fluid and/or the analyte to the first electrode (101) via the at least one opening (107).

FIG. 1a, 110

EP 4 278 966 A1

## Description

### State of the art

The invention relates to an electrochemical analyte sensor for measuring analyte data, e.g. glucose data, in an analyte carrying fluid.

Electrochemical analyte sensors exploit the fact that certain electrochemical reactions of an analyte to be detected or to be measured can generate electrical signals, e.g. electric currents or electric potentials, that can correlate with the concentration of the analyte to be detected, e.g. that are directly or indirectly proportional to the concentration of the analyte.

For example, a potentiometric electrochemical analyte sensor can generate electric potentials that can exhibit a logarithmic dependence on the concentration of an analyte to be detected.

For example, an amperometric electrochemical analyte sensor can generate an electric current that can flow between electrodes of the sensor that can be proportional to the concentration of the analyte to be measured.

Such analyte sensors are, for example, commonly used for measuring glucose levels, and in particular can be part of in-vivo continuous glucose monitoring systems that measure glucose levels in the blood and/or in interstitial fluids of patients suffering from diabetes.

However, current electrochemical analyte sensors suffer from numerous drawbacks. For example, electrodes of current analyte sensors can exhibit significant material wear and can leak undesired chemicals or materials into their environment.

Furthermore, current electrochemical analyte sensors may suffer from slow response times and can exhibit non-linear measurement characteristics that can significantly degrade the accuracy, robustness and reliability of analyte measurements.

In addition, the structure and design of current electrochemical analyte sensors are rather complex and intricate, and require complicated and costly manufacturing steps.

### Problem

It is therefore an objective of the present invention to provide means for obtaining more robust, more accurate and more reliable analyte measurements, e.g. glucose measurements, using an electrochemical analyte sensor.

It is further an objective of the present invention to provide an improved electrochemical analyte sensor.

In particular, it is an objective to provide a simplified, more compact, more cost-effective, more precise and more reliable electrochemical analyte sensor.

### Solution

According to the present invention, at least one of said objectives is achieved by the subject-matter of the independent claims.

Advantageous embodiments and further developments are the subject-matter of the subclaims.

Further advantageous embodiments and further developments are disclosed throughout the specification.

For example, an electronic analyte sensor for use in medical devices for measuring analyte data in an analyte carrying fluid, in particular for measuring glucose data, may comprise one, some or all of the following components:

- a first electrode, said first electrode being a working electrode
- a second electrode,
- a substrate (100) carrying the first electrode and the second electrode,
- at least one membrane which at least partially covers the first electrode,
- wherein the at least one membrane comprising a membrane material that is impermeable to the analyte carrying fluid and/or the analyte, wherein the at least one membrane comprises at least one opening, and wherein the at least one membrane is configured for controlling the flux of the analyte and/or the flux of the analyte carrying fluid to or towards the first electrode, in particular towards an electrochemically active layer of the first electrode, via the at least one opening.

The at least one opening or a plurality of openings of the at least one membrane provide(s) a well-defined flux channel for the analyte and/or for the flux of the analyte carrying fluid to the first electrode / the working electrode, in particular to a part of the working electrode that comprises electrochemical conversion agents, e.g. enzymes and/or catalysts, i.e. to an electrochemically active layer of the working electrode.

The at least one membrane may also be referred to as flux localization membrane.

The working electrode, for example, may comprise a sensing material or sensing layer as electrochemically active layer that is configured to provide at least one enzyme to the first electrode / the working electrode.

Exemplary properties of such a sensing material or sensing layer are described further below.

The at least one membrane can at least partially cover the first electrode and optionally can also cover the second electrode. However, the at least one opening / the plurality of openings of the at least one membrane can be only located in the part of the membrane that at least partially covers the first electrode.

Herein the expression "cover" can include also the case that additional layers or components are present between the at least one membrane and the first electrode and/or the second electrode, i.e. the expression "cover" does not necessarily imply a direct contact between the at least one membrane and the first electrode and/or the second electrode.

The analyte sensor or electrochemical analyte sensor can, for example, be an amperometric analyte sensor or a coulometric analyte sensor.

The analyte sensor can be configured for being used in medical devices, in particular for being used in portable, e.g. wearable / on-body, electronic medical devices that can measure analyte data and other medical data.

The substrate for carrying or supporting the first electrode and the second electrode and further components of the analyte sensor can be a flat and planar structure and the substrate material may comprise polyester.

The first electrode and the second electrode of the analyte sensor can be arranged on opposing sides or opposing surfaces of the substrate, e.g. the top surface and the bottom surface. However, it is also conceivable that the first electrode and the second electrode are arranged on the same side or same surface of the substrate in a coplanar manner.

Herein the analyte carrying fluid can inter alia be understood as referring to body fluid, e.g. blood and/or interstitial fluids, of a user, in particular of a patient suffering from diabetes.

The analyte sensor may further comprise at least one sensor circuit connected to the electrodes and that can be configured for operating and controlling the electrodes and the analyte sensor operations. Furthermore, the analyte sensor may comprise a power source, e.g. a battery, that can provide power to analyte sensor, in particular to the sensor circuit of the analyte sensor.

However, it is also conceivable, that in addition or alternatively, the analyte sensor can be configured to draw power from electrochemical reactions occurring at the electrodes during use of the analyte sensor, i.e. when the electrodes of the analyte sensor are in contact with a body fluid of a user.

Herein, said membrane that is impermeable to the analyte or analyte carrying fluid may also refer to a component comprising one or more layers.

Said membrane or membrane layers can further be understood as acting as a selective barrier, in particular as a locally selective barrier, that allows only specific particles / molecules / compounds / fluids / fluid components to pass, whereas other particles / molecules / compounds / fluids / fluid components are stopped by the membrane, i.e. cannot diffuse through the membrane or can diffuse through the membrane only to a small / negligible extent.

In particular, the membrane material may be hydrophobic.

The membrane material can inter alia also be an electric isolator material or a semiconductor material.

The term impermeable membrane or impermeable membrane material can herein in particular be understood as referring to a membrane / membrane material that is essentially impermeable to specific particles, molecules, compounds, fluids, or fluid components, i.e. the membrane or membrane material can be understood as having a low permeability for specific particles, molecules, compounds, fluids or fluid components.

However, the term impermeable membrane or impermeable membrane material, aside from referring to a membrane or membrane material that is fully, e.g. 100%, impermeable for the analyte or analyte carrying fluid, may also comprise the case of the impermeable membrane or impermeable membrane being only sufficiently impermeable, e.g. blocking only at least 60% or 80% or 90% or 99% of the analyte or analyte carrying fluid.

For example, said exemplary membrane comprising a membrane material that is impermeable to the analyte or the analyte carrying fluid can be understood as a membrane comprising a membrane material that has an effective diffusion coefficient for the analyte carrying fluid that is at least one or two orders of magnitudes smaller than the diffusion coefficient of a membrane material that is permeable or fully permeable to the analyte carrying fluid.

In other words, the at least one membrane or membrane material can provide an analyte flux insulation, i.e. the at least one membrane can limit the flux of analyte or of the analyte carrying fluids to components or layers below the membrane or membrane material.

For example, the at least one membrane or membrane material can be configured such that at least 90%, preferably more, of the total flux of the analyte or the analyte carrying fluid through the membrane is carried only by the opening(s) in the membrane.

Further exemplary properties of the membrane material that is impermeable to the analyte or analyte carrying fluid are described further below.

As mentioned above, the at least one membrane can comprise at least one opening, i.e. one or more openings or a plurality of openings.

An opening may herein be inter alia understood as a hole in or through the membrane, e.g. a through hole or bore hole, a perforation and/or a slot or slit in the membrane, thereby allowing the passage of one or more particles or molecules and/or compounds or fluid components through the opening and through the membrane.

The at least one opening may extend through or penetrate completely through the membrane or membrane material. The at least one opening may extend also through other layers below the membrane.

In particular, the at least one opening allows the passage of the at least one analyte, e.g. glucose, to be measured and, more particularly, allows the passage of analyte carrying fluid, e.g. body fluid, which comprises the at least one analyte to be measured, to an electrochemically active layer or sensing layer of the working electrode and/or to other electrodes.

Stated differently, the at least one opening of the at least one membrane provides access for the analyte / the analyte carrying fluid to the / to an electrochemically active layer or sensing layer of the working electrode. Exemplary properties of said electrochemically active layer or sensing layer or sensing material layer are described further below.

In other words, via the opening or via the plurality of openings of the at least one membrane, the flux of the analyte carrying fluid to an electrode of the analyte sensor, e.g. the flux or flux transport of the analyte / analyte carrying fluid to a/the working electrode or to a/the counter electrode of the analyte sensor can be controlled / regulated.

Herein the term or act of controlling or regulating the flux of the analyte or the analyte carrying fluid can inter alia refer to increasing and/or decreasing or limiting or localizing the flux of the analyte or the analyte carrying fluid towards an electrode of the analyte sensor, e.g. towards the first electrode, e.g. the working electrode and/or the second electrode, e.g. the counter electrode.

The at least one opening in the membrane may, for example, be generated or created or formed by drilling, e.g. with a laser, e.g. by laser ablation, or with other drilling tools.

It can be preferred to use a laser ablation process for creating or forming the opening in the membrane, as this can allow a better, more accurate, control of the properties, e.g. geometry and/or surface roughness, of the opening and its wall surface(s) in the membrane.

However, it is also conceivable to use a self-organizing material for the membrane material, e.g. copolymers of immiscible monomers that form the desired morphology of the membrane. Also the use of a photoresist material is conceivable.

The above and herein exemplary described structure of the analyte sensor provides numerous advantages. In particular, the improved control of the flux towards the electrodes, e.g. towards the working electrode and/or towards the counter electrode, can, for example, prevent the occurrence of flux overload to the working electrode, i.e. can prevent that an electrochemical conversion agent of the analyte sensor electrode(s), e.g. an enzyme and/or catalyst, is overloaded.

The analyte sensor can therefore be operated in an analyte-flux-transport-controlled manner, i.e. in an analyte-flux-transport-controlled regime. In other words, it can be avoided that the analyte sensor operates in regimes that exhibit severe non-linear measurement behaviors, such as when the analyte sensor operates in a kinetic, i.e. reaction, controlled regime.

Furthermore, this facilitates that the analyte transport or analyte flux becomes rate determinant and thus the reaction current has a linear or substantially linear relation to the concentration of the analyte to be measured. Hence, being able to work in a flux-transport-controlled regime can improve the accuracy and reliability of analyte data measurements of the analyte sensor.

Moreover, the above and herein exemplary described structure of the analyte sensor can ensure that the advantageous linear measurement characteristics of the analyte sensor are maintained independent of external factors such as temperature and humidity and also independent of possible enzyme activity changes at the working electrode, i.e. in a / at the sensing layer of the working electrode, e.g. due to aging.

The above and herein exemplary described structure of the analyte sensor further allows that a sufficient or large amount of electrochemical conversion agents, e.g. enzymes and/or catalysts, are always available in close proximity of / at a sensing layer of an electrode of the analyte sensor, e.g. at the working electrode.

This way, the local analyte flux density can be increased without leaving the global analyte-flux-transport-controlled regime.

In addition, the at least one opening of the at least one membrane can provide a rather high analyte flux throughput / analyte carrying fluid flux throughput to the electrode(s), e.g. the working electrode, of the analyte sensor.

The locally high analyte flux density can inter alia ensure that the analyte sensor has a fast response time, e.g. in the order of tens of seconds or less.

In other words, the above and herein exemplary described analyte sensor has advantageous linear measurement characteristics and advantageously fast response times in comparison with state-of-the-art analyte sensors.

The shape(s) / cross section(s) of the at least one opening(s) can be round, substantially round, oval, polygonal, rectangular, square, regular, irregular, slit-like.

In the exemplary case of the membrane comprising a plurality of openings, the shape(s) / cross section(s) of the openings of the plurality of openings can be the same or each of the openings can have a different shape or cross sections or subsets of the plurality of openings can have different shapes or different cross sections between different subsets, i.e. the plurality of openings can be grouped into subsets of openings having the same or different shapes or cross sections.

An exemplary diameter or mean diameter of an opening / the openings can be of similar size to the thickness of the at least one membrane and can, for example, lie in the range of 10 to 100 micrometers.

For example, all openings may have a similar or equal shape and size.

Furthermore, the openings may be arranged on a regular grid with a homogeneous density or on a randomized grid with a homogeneous density.

The distance between adjacent or neighboring openings can be significant larger, e.g. at least an order of magnitude larger, than the thickness of the membrane or the thickness of any other layers of the analyte sensor.

This further facilitates ensuring that the analyte sensor can work in an optimal linear analyte-flux-transport-controlled manner / regime.

The more openings and the further the separation of adjacent openings, the better the analyte flux localization and control and the more robust the measurements of the analyte sensor.

The above and herein described openings of the at least one membrane, i.e. the flux localization membrane, can provide a plurality of discrete, separate linear, e.g. straight, flux channels through the membrane.

For example, adjacent streams of analyte or analyte carrying fluid that pass through the membrane via adjacent openings do not converge after having passed through the membrane but leave the membrane as separate streams.

This behavior is inter alia different from the behavior of fluid passing through pores of a sponge like structure, wherein fluid streams from different pores merge before and / or immediately after having passed through the pores of the sponge like structure.

In other words, the opening(s) of the at least one membrane cannot be / are not to be equated with pores.

The opening(s) of the at least one membrane is / are fully permeable to the analyte / to the analyte carrying fluid and can be filled at least partially or fully with a material that is permeable to the analyte carrying fluid, e.g. a hydrophilic polymer.

Said filling material can be medium to highly penetrable / permeable by the analyte / analyte carrying fluid.

For completeness, it is noted that the filling material can also be air.

As indicated earlier, the opening(s) and the filling material of the opening(s) can be configured such that at least 90%, preferably at least 99.9% of the total analyte flux passing through the membrane, passes through the opening(s) in the membrane.

It is also possible that the at least one membrane is embedded in said filling material that is permeable to the analyte / analyte carrying fluid. Stated differently, said filling material can form a filling material layer or diffusion limiting layer, that allows to control the diffusion of the analyte or the analyte carrying fluid through the opening(s) of the membrane, i.e. the flux localization membrane.

Said filling material or said diffusion limiting layer that is permeable to the analyte / analyte carrying fluid can fully or at least partially cover the first electrode (101) and/or the second electrode.

Further properties of the filling material or the diffusion limiting layer are described further below.

The above and herein exemplary described structure of the analyte sensor can provide a spatial modulation of the analyte flux within or across the analyte sensor, wherein locally a very high analyte flux density/ flux rate can be achieved through (and above or below) the opening(s) of the membrane and a negligible analyte flux or analyte flux rate can occur in between said openings or far from said openings.

The response time characteristic of the analyte sensor can then be given by the transport characteristics of the opening(s) alone (flux-weighted average) and can thus be extremely fast as compared to current sensors and response times of less than a few tens of seconds can be achieved.

The above-mentioned first electrode can be a working electrode, i.e. the at least one membrane can be configured for controlling the flux of the analyte / the analyte carrying fluid to the working electrode via the opening(s).

Exemplary properties of the sensing material / sensing layer of the working electrode

To enable/generate and facilitate an/the electrochemical reaction of the analyte sensor with an analyte to be measured, the working electrode can comprise electrochemical conversion agents, e.g. enzymes and/or catalysts.

The analyte sensor can, for example, comprise a sensing material or sensing material layer or sensing layer as an electrochemically active layer that is configured to provide at least one enzyme to the first electrode / the working electrode. Said exemplary sensing layer may be located / arranged below / beneath the at least one membrane.

In particular, the first electrode / the working electrode can comprise or can be covered by a sensing material or sensing layer comprising at least one enzyme.

Stated differently, the sensing material or sensing layer can be part of the working electrode.

The first electrode / the working electrode may comprise only one enzyme or a mixture of two or more enzymes. Only one enzyme is preferred. Specifically, the enzyme is capable of catalyzing a chemical reaction converting the analyte, in particular glucose. Even more specifically, the at least one enzyme is selected from the group consisting of a glucose oxidase (EC 1.1.3.4), a hexose oxidase (EC 1.1.3.5), an (S)-2 hydroxy acid oxidase (EC 1.1.3.15), a cholesterol oxidase (EC 1.1.3.6), a glucose dehydrogenase, a galactose oxidase (EC 1.1.3.9), an alcohol oxidase (EC 1.1.3.13), an L-glutamate oxidase (EC 1.4.3.11), and an L-aspartate oxidase (EC 1.4.3.16). In particular, the at least one enzyme is a glucose oxidase (GOx) and/or modifications thereof. In another preferred embodiment, the at least one enzyme is a glucose dehydrogenase, in particular an FAD-dependent glucose dehydrogenase (FAD-GDH).

Said at least one enzyme may be comprised in said exemplary sensing material or in the sensing layer covering the first electrode / the working electrode. The sensing material or sensing layer may further comprise at least a polymeric material; specifically, it may be or may comprise at least a polymeric material and at least a metal containing complex. The metal containing complex may be selected from the group consisting of transition metal element complexes; specifically, the metal containing complex may be selected from osmium-complexes, ruthenium-complexes, vanadium-complexes, cobalt-complexes, and iron-complexes, such as ferrocenes, such as 2-ami-noethylferrocene. Even more specifically, the sensing material may be a polymeric transition metal complex as described for example in WO 01/36660 A2, the content of which is included by reference. In particular, the sensing material may comprise a modified poly(vinylpyridine) backbone loaded with poly(bi-imidizyl) Os complexes covalently coupled through a bidentate linkage. A suitable sensing material is further described in Feldman et al, Diabetes Technology & Therapeutics, 5 (5), 2003, 769-779, the content of which is included by reference. Suitable sensing materials further may include ferrocene-containing polyacrylamide-based viologen-modified redox polymer, pyrrole-2,2'-azino-bis(3-ethylbenzthiazoline-6-sulfonic acid)(ABTS)-pyrene, Naphthoquinone-LPEI. The polymeric transition metal complex may represent a redox mediator incorporated into a cross-linked redox polymer network.

This is advantageous as it may facilitate electron transfer between the at least one enzyme or analyte and the conductive trace that connects the working electrode with the sensor electronics.

In order to avoid or reduce unwanted elutriation or elution processes, the redox mediator and the enzyme may be covalently incorporated into a polymeric structure.

In an exemplary embodiment, the sensing material or sensing layer may comprise a polymeric material and $MnO_2$-particles or any other material catalyzing hydrogen peroxide oxidation reaction as well as the at least one enzyme. Another possible material catalyzing hydrogen peroxide oxidation reaction is Pt (platinum).

Moreover, the sensing material may additionally comprise at least one crosslinker; the crosslinker may for example be capable of crosslinking at least part of the sensing material.

Furthermore, the first electrode / working electrode of the analyte sensor may comprise carbon / graphite and other $sp^2$ hybridized carbon allotropes as conductive material.

The exemplary sensing material or sensing layer of the first electrode / the working electrode can be located below the at least one membrane / below the at least one opening of the at least one membrane and can inter alia extend over the large parts or over the whole surface of the substrate onto which the working electrode can be arranged.

In other words, the structure of the herein exemplary described analyte sensor can be a multi-layer structure which comprises several layers / structures / coatings.

For example, the part of the analyte sensor comprising the first electrode, i.e. the working electrode, may be structured with multiple layers as follows, wherein the order is specified with reference to the outside environment towards the inside of the analyte sensor, i.e. towards the substrate of the analyte sensor:

- at least one membrane comprising a membrane material that is impermeable to the analyte carrying fluid and/or the analyte, wherein the at least one membrane comprises at least one opening,
- at least one sensing layer,
- a first electrode being configured as working electrode,
- a substrate, wherein the substrate is configured for carrying and/or supporting the above identified components.

The at least one sensing layer may be part of the working electrode.

As previously indicated, the opening(s) / of the at least one membrane may be filled at least partially or fully with a material that is permeable to the analyte carrying fluid, e.g. a hydrophilic polymer. This filling material can serve to control the flux density and/or the diffusion of the analyte carrying fluid / analyte passing through the opening(s) of the at least one membrane.

The at least one membrane may be embedded in said filling material. In other words, the at least one membrane may lie below / beneath a layer of filling material that is permeable to the analyte carrying fluid, e.g. a hydrophilic polymer layer.

Hence, said filling material can be understood as an additional layer in the above exemplary described structure, wherein a filling material layer can be arranged above the at least one membrane and/or below the at least one membrane and/or can embed the at least one membrane.

Stated differently the filling material can form a layer that is arranged on top of any other possible layers present in the analyte sensor and/or the filling material can form a layer that is arranged between other layers on the analyte sensor. The filling material or the filling material layer can also be referred to as a diffusion limiting layer or as a flux limiting layer, since as mentioned above, it can serve to control the flux density and/or the diffusion of the analyte carrying fluid / analyte passing through the opening(s) of the at least one membrane.

For completeness, it is further noted that while the analyte carrying fluid, which can also be referred to as the solvent, can be hydrophobic, the analyte carrying fluid can also be more susceptible to the diffusion limiting layer, i.e. the analyte carrying fluid can pass the diffusion limiting layer more easily. In contrast thereto the analyte, which can also be referred to as the solvate, can be less susceptible to the diffusion limiting layer and thereby the diffusion limiting layer can further control / limit the diffusion of the analyte through the opening(s) of the at least one membrane.

Hence, a herein described exemplary analyte sensor may be configured such that below / beneath the at least one membrane (with the at least one opening) and below a layer filling material can lie a thin layer of sensing material / a thin sensing layer, wherein said sensing layer can be part of the working electrode or can be a layer in direct contact with the working electrode.

Herein a thin sensing layer can be understood as referring to a layer thickness in the range of a few micrometers, e.g. less than 5 μm.

Due to the thinness of the sensing layer it can be ensured that a large / sufficient amount of electrochemical conversion agents, e.g. enzymes and/or catalysts, is available in close proximity of / at the first electrode / the working electrode.

Furthermore, the sensing layer can have a permeability to the analyte or to the analyte carrying fluid that is as least as high as, preferably higher / substantially higher than, the permeability of the optional filling material of the opening(s).

This can facilitate the interaction of the analyte / analyte carrying fluid with the sensing layer of or associated with the first electrode / the working electrode.

For example, due to the high permeability to the analyte / analyte carrying fluid of the sensing layer, the analyte / analyte carrying fluid / analyte flux that is located at / below the opening(s) can reach also parts / locations of the sensing layer / of the first electrode / the working electrode that are far / further away from the location of the opening(s) of the membrane.

This further facilitates the realization of an analyte sensor that operates in a reliable, accurate manner in a linear measurement regime and that has fast response times.

For completeness, it is noted that the possible layers beneath or above the membrane can also comprise openings that are aligned with the opening(s) of the membrane. For example, the sensing layer and/or the electrode, e.g. a/the first electrode, e.g. a/the working electrode, and/or a/the second electrode, e.g. a/the counter electrode, may have an opening / may have openings that are aligned with the opening(s) in the membrane layer.

Furthermore, it is noted that the said optionally exemplary filling material layer or diffusion limiting layer can have a thickness of a few micrometers, e.g. less than 5 μm.

The at least one membrane comprising a membrane material that impermeable to the analyte carrying fluid and/or the analyte can also have a thickness of a few micrometers, e.g. less than 5 μm, and even down to one or a few nanometers.

The first electrode / working electrode can have also have of a few micrometers, e.g. less than 5 μm, and even down to one or a few nanometers.

In an alternative exemplary multi-layer structure design of the part of the analyte sensor comprising the first electrode, i.e. the working electrode, the multiple layers can be structured as follows, wherein the order is again specified with reference to the outside environment towards the inside of the analyte sensor, i.e. towards the substrate of the analyte sensor:

- a filling material layer with a material that is permeable to the analyte / analyte carrying fluid, e.g. a hydrophilic polymer
- a first electrode being configured as a working electrode, wherein the first electrode comprises a material that is impermeable to the analyte and/or the analyte carrying fluid and is configured to act as a locally selective barrier for the analyte and/or the analyte carrying fluid, wherein the first electrode / the working electrode comprises at least one opening, to allow the analyte / analyte carrying fluid to pass through towards at least sensing layer,
- at least one sensing layer that is part of the first electrode / the working electrode or that is associated to the first electrode / the working electrode,
- a substrate wherein the substrate is configured for carrying and/or supporting the above identified components.

In this exemplary alternative design, the first electrode / the working electrode itself acts as a membrane that comprises a material that is impermeable to the analyte / analyte carrying fluid and is configured to allows a passage of the analyte / analyte carrying fluid only through the at least one opening of the first electrode / the working electrode.

For example, said electrode material that is impermeable to the analyte / analyte carrying fluid can comprise carbon.

Hence, and alternative design of an analyte sensor for use in medical devices for measuring analyte data in an analyte carrying fluid, in particular for measuring glucose data may comprise:

a first electrode being a working electrode, and

a second electrode,

a substrate carrying the first electrode and the second electrode,

wherein the first electrode comprises a material that is impermeable to the analyte carrying fluid and/or impermeable to the analyte, and wherein the first electrode comprises at least one opening, and wherein the first electrode is configured for controlling the flux of the analyte carrying fluid and/or the analyte to an electrochemical active layer of the first electrode via the at least one opening.

Said electrochemical active layer or sensing layer of the first electrode can correspond to or be equal to the electrochemical active layer or sensing layer and its properties as described with respect to the designs comprising the flux localization membrane.

As in the designs that comprise a flux localization membrane with openings, this alternative design can also comprise a diffusion limiting layer / filling material layer that is permeable to the analyte / to the analyte carrying fluid and which can fully or at least partially fill the openings of the first electrode / the working electrode.

Also said diffusion limiting layer / filling material layer can fully or at least partially cover the first electrode / the working electrode and/or the second electrode / the counter electrode.

The opening(s) of the first electrode / the working electrode in this example, can have the same properties, e.g. the same geometry and dimensions, as the opening(s) of the above described at least one membrane that comprises a membrane material that is impermeable to the analyte / analyte carrying fluid.

To ensure the possible alignment of the possible openings in all possible designs of the analyte sensor and across all or some of the possible different layers of the analyte sensor, said possible openings may be created / generated / formed via the same process, e.g. via laser ablation or another drilling method.

The above-mentioned exemplary second electrode can be an electrode selected from a group comprising the following types: a counter electrode and a combined counter/reference electrode.

Unless explicitly specified otherwise, the herein described first electrode can be referred to as working electrode and the second electrode can be referred to as counter electrode or as combined counter/reference electrode of the herein described analyte sensor.

Furthermore, the second electrode / the counter electrode can be an oxygen electrode that can be configured to use oxygen solved/dissolved in the analyte carrying fluid / body fluid / interstitial fluid as electron acceptor and can comprise gold or platinum as conductive material and catalyst.

Using oxygen solved in the analyte carrying fluid as a renewable source, the wear time of the second electrode / the counter electrode can be increased / extended over current state-of-the-art counter electrodes.

Furthermore, the herein described analyte sensor design allows dispensing with the need for a dedicated reference electrode, in particular reference electrodes that are prone to undesired material wear and that can leak undesired material into their environments, such as, for example, Ag/AgCl (silver / silver chloride) reference electrodes. This can inter alia further improve the lifetime of an analyte sensor.

The second electrode can further be configured to measure an oxygen saturation / oxygen saturation level / oxygen saturation concentration in its environment, i.e. in the analyte carrying fluid / body fluid / interstitial fluid.

Such an optional measurement of an oxygen saturation / oxygen saturation level / oxygen saturation concentration can be used to compensate for possible measurement errors of the analyte sensor, since the oxygen saturation / oxygen saturation level / oxygen saturation concentration can influence the electrochemical reaction used by the analyte sensor to measure the analyte concentration, in particular in the case of oxidative electrochemical reactions.

The optional possibility to measure and output an oxygen saturation / oxygen saturation level / oxygen saturation concentration in the body fluid, e.g. blood and/or in interstitial fluids, of patients may also be useful for further additional therapeutic and diagnostic purposes.

As exemplary described above, the analyte sensor may comprise a substrate, wherein the first electrode, e.g. the working electrode, and the second electrode, e.g. the counter electrode, can be arranged / located on different sides / different locations / different parts / different portions of a/the surface of the substrate.

The substrate can be a flat and planar structure, wherein, for example, the first electrode / the working electrode can be arranged on a top surface of the substrate and the second electrode / the counter electrode can be arranged on the bottom surface of the substrate.

However, it is also conceivable, that the electrodes can be arranged in a coplanar manner on the surface of the substrate at different locations on the same surface of the substrate / substrate layer.

Furthermore, the electrodes, i.e. both the first electrode / the working electrode and the second electrode / the counter electrode, in all herein described variants and embodiments can be planar electrodes.

In addition to the electrodes, the substrate may further be configured to carry any sensor circuit components in connection / in association with the electrodes and further components of the analyte sensor, e.g. a power source that can provide power to a/the sensor circuit of the analyte sensor.

Hence, the above exemplary described multilayer structure of the analyte sensor can include a substrate / substrate layer below / beneath an electrode, e.g. beneath the first electrode / working electrode and/or the second electrode / counter electrode.

An exemplary design of the analyte sensor with electrodes arranged on opposing / different sides of a/the substrate, i.e. on opposing substrate surfaces, can enable a more compact analyte sensor and more cost efficient manufacturing, since it is not necessary to have the conductive traces for both electrodes on the same side.

For example, it is possible to cover the first side of the substrate with the conductive material of the first electrode / working electrode and the second side of the substrate with the conductive material of the second electrode / counter electrode and then apply further layers of materials, if necessary.

Said exemplary substrate / substrate layer that can accommodate the electrodes and the sensor circuit and other components of the analyte sensor may, for example, comprise polyethylene terephthalate (PET).

However, it is conceivable that other electrode designs may be implemented for the analyte sensor, e.g. non-planar designs such as cylindrical / rod-like electrodes.

The at least one membrane can also be configured for controlling the flux of the analyte / analyte carrying fluid to the second electrode, e.g. a/the counter electrode.

In the exemplary case of the first electrode and the second electrode being arranged in a coplanar manner on different / opposing sides of a/the substrate / substrate layer, the at least one membrane may be configured for covering both electrodes at the same time.

Alternatively, it is possible that the analyte sensor comprises two membranes, a first and a second membrane, wherein the first membrane is configured to cover the first electrode, e.g. a/the working electrode, and the second membrane is configured to cover the second electrode, e.g. a/the counter electrode.

Therein, the membrane(s) can be applied, for example, by dip coating, to the first electrode, e.g. a/the working electrode, and/or to the second electrode, e.g. a/the counter electrode.

As previously indicated, the analyte sensor can comprise at least one sensor circuit that comprises / is connected with the electrodes and that can be configured for operating and controlling the operations of the analyte sensor and its electrodes.

As also previously indicated, the analyte sensor may comprise a power source, e.g. a battery, that can provide power to analyte sensor, in particular to the sensor circuit and/or the electrodes of the analyte sensor.

The sensor circuit may inter alia further comprise at least one capacitor.

Said possible exemplary capacitor can be used during operation of the analyte sensor to integrate an electrical signal / electrical current flowing between the electrodes, i.e. between the first electrode / working electrode and the second electrode / counter electrode, of the analyte sensor.

Said integration can then yield a capacitor-charge-proportional voltage signal that can be used to determine analyte data and/or oxygen data in the environment of the analyte sensor, e.g. in a body fluid.

For example, measuring the current I from the enzymatic glucose conversion times a predetermined time increment $\Delta t$, i.e. integrating the current I over time, yields the charge Q stored on the capacitor, which is proportional to the capacitor voltage V (at least for lower voltage levels).

Hence, the following relation

$$I * \Delta t = Q \sim V$$

can be exploited to determine analyte data, e.g. glucose concentration, since the measured glucose current I and thus the glucose concentration is proportional to the capacitor voltage increase in a given time interval.

Herein, said exemplary lower voltage levels may refer to voltages of less than 0.5 V or less than 0.4 V and the exemplary current integration time can be based on the order the sampling frequency of the analyte sensor, which can be every minute or every few minutes.

Data on the oxygen level and its diffusion flux can be obtained on the basis of the Nernst equation, which can, for example, be expressed as

$$E_{CE} = E_{O_2}{}^0 + \frac{R\,T}{z\,F} \log\{c_{O_2}\}$$

, wherein $E_{CE}$ is the electrode potential of the counter electrode, and wherein the upper index 0 denotes given standard conditions (i.e. temperature and pressure) of the standard oxygen electrode potential $E_{O_2}{}^0$, R is the universal gas constant, T is temperature, z is the charge transfer number, F is the Faraday constant and $\log\{c_{O_2}\}$ is the natural logarithm of the oxygen concentration $c_{O_2}$ in units mol/l.

However, also other variants of the Nernst equation may be used, which inter alia account for partial pressure and activity that may change the standard potential.

Time oscillations of the electrode voltage, i.e. the potential difference between the electrodes of the analyte sensor, i.e. between the potential of the working electrode $E_{WE}$ and the potential of the counter electrode $E_{CE}$, are governed by the oxygen availability and thus allows to calculate the dynamic flux of oxygen. Hence, the overall oxygen level can be obtained from the average (or maximum) electrode voltage.

Said electrical signal / electrical current flowing between the electrodes can thereby be generated by applying, by the sensor circuit of the analyte sensor, a voltage signal between the first electrode and the second electrode of the analyte sensor.

An exemplary method for manufacturing the above and herein described exemplary analyte sensor for use in medical devices for measuring analyte data in an analyte carrying fluid, in particular for measuring glucose data, can therefore comprise one, some or all of the following steps:

- mounting a sensor circuit comprising a first electrode, e.g. a working electrode, and a second electrode, e.g. a counter electrode, onto a substrate, wherein the first electrode and the second electrode can be mounted on different sides, e.g. opposing sides, e.g. top and bottom surface sides, of the substrate or on the same side,

- applying at least at one membrane comprising a membrane material that is impermeable to the analyte carrying fluid and covering at least partially or fully the first electrode and optionally the second electrode with the at least one membrane.

Before or after the mounting of the sensor circuit and electrodes on the substrate, a sensing layer, as described further above, can be applied to the electrodes, e.g. to the first electrode / to the working electrode.

This step can be carried out before the at least one membrane is applied such that the membrane can, for example, cover at least partially or fully the sensing layer and the underlying first electrode / working electrode.

As previously mentioned, it is also conceivable that the sensing layer is already part of the first electrode / the working electrode and does not have to be applied to the first electrode / the working electrode after the first electrode / the working electrode is mounted to the substrate.

Before or after applying the at least at one membrane, at least one opening can be formed / created / generated in the membrane.

For example, the at least one opening may be formed / created / generated by a drilling and/or ablation process, e.g. by laser ablation.

After applying the at least one membrane, the at least one opening can be filled fully or at least partially with a (the previously mentioned) material that is permeable to the analyte carrying fluid, e.g. a hydrophilic polymer.

It is also possible that the at least one membrane is embedded in said filling material that is permeable to the analyte carrying fluid and/or a layer of said filling material is applied onto / on top of the at least one membrane and the at least one opening.

The filling material or filling material layer or diffusion limiting layer can also be applied to cover the second electrode / the working electrode.

The above exemplary described steps of applying a membrane or a layer can be carried out via dip coating

The step of forming / creating / generating the at least one opening can also be carried out as a last step i.e. after all other components and layers of the analyte sensor have been mounted onto the substrate.

This can inter alia allow to form the at least one opening in the at least one membrane with different depths across different layers / different materials of the analyte sensor.

For example, in an exemplary first type of the at least one opening, the opening may be formed such that it penetrates / extends only through the at least one membrane and not to any possible further layers of the analyte sensor below the membrane.

In an exemplary second type of the at least one opening, the opening may be formed such that it penetrates / extends through the at least one membrane and at least partially or fully further extends to / extends through a/the first layer below the membrane, e.g. a/the sensing layer or a/the filling material layer.

In an exemplary third type of the at least one opening, the opening may be formed such that it penetrates / extends through the at least one membrane and further extends through any other layer below the membrane including extending partially of full through an/the electrode lying below the membrane, e.g. through the first electrode / the working electrode.

It is also even possible that the opening extends partially through the substrate.

In other words, the depth of the at least one opening can vary and in the described exemplary types can increase from the exemplary first type to the exemplary third type.

This further can facilitate optimizing the control of the analyte flux / analyte carrying fluid to the electrodes of the analyte sensor, e.g. to the first electrode / the working electrode.

Furthermore, it is conceivable that the at least one membrane is applied / configured such that its possible edges / borders / lateral sides can wrap at least partially or fully around the edges / borders / lateral sides of the layers and/or components lying below the at least one membrane.

For example, in a first type of an edge of the at least one membrane, the edge may be configured such as to wrap in an L-shaped manner, e.g. with the long side of the L being parallel to the substrate and with the short side of the L being orthogonal to the substrate, around the first layer below the membrane, e.g. a/the sensing layer or a/the filling material layer.

In an exemplary second type of an edge of the at least one membrane, the edge may be configured such as to wrap in said L-shaped manner around all layers / all components below the membrane, e.g. a/the sensing layer and a/the filling material layer and an electrode, e.g. first electrode / the working electrode, until making contact with the substrate.

Said edge types and opening types are exemplary only and other types are conceivable too.

For example, the L-shape of an edge is not restricted to the long side or the short side of the L being linear straight sides, but said sides can be curved or can be adapted to the shape of the cross-sections of the various layers, including non-rectangular cross sections.

As indicated further above, the analyte sensor can comprise a sensor circuit that, aside from the electrodes and other possible electronic components, may further comprise at least one capacitor.

An exemplary method of operating a herein described analyte sensor may comprise one, some or all of the following steps:

- applying a voltage signal between the first electrode / working electrode and the second electrode / counter electrode of the analyte sensor

- integrating an electrical signal, e.g. an electrical current, between first electrode and the second electrode via at least one capacitor, wherein said electrical signal was generated in response to an electrochemical reaction of the first electrode with an analyte or analyte carrying fluid, said integration generating a voltage signal that is proportional to the charge of the at least one capacitor,

- measuring analyte data based on the generated voltage signal.

Said electrochemical reaction of the first electrode with an analyte or analyte carrying fluid can occur in the above describe electrochemically active layer or sensing layer of the first electrode.

The step of applying a voltage signal inter alia can comprise applying the voltage externally, i.e. from a voltage source. However, this step can also comprise using the voltage / current produced by voluntary electrochemical reactions.

The upbuilding integrating voltage then slowly reduces the current until it becomes zero.

An externally triggered shorting (or measurement or over a resistor) can discharge the capacitor.

This can be combined with an opening of the sensor circuit to stop the electrochemical reaction. During the reaction stop step? the oxygen level can recover which changes the open circuit potential (OCP), while no current is flowing.

Stated differently, the step of applying a voltage signal can also comprise applying or carrying out open circuit potential operations.

Evaluating the OCP time function (e.g. at the start and end of the interruption of the electrochemical reaction) allows to calculate the desired oxygen level and flux.

In addition to analyte data, such as a concentration of an analyte, e.g. a glucose concentration, other data can be measured based on the generated voltage signal as well, such as oxygen data.

Said oxygen data may, for example, include oxygen saturation / oxygen saturation level / oxygen saturation concentration in the body fluid / analyte carrying fluid, e.g. in blood or interstitial fluid, of a patient or user.

The measurement / determination of said data, e.g. analyte data and/or oxygen data, from said generated voltage signal is possible with high accuracy and reliability, since the generated voltage signal is proportional to said data and due to the fact that the herein described analyte sensor has good linear measurement characteristics.

The exemplary method may further comprise a step of periodically discharging the capacitor over a known resistor from which the capacitance is determined.

This can inter alia ensure that the electrochemical potential difference between the electrodes of the analyte sensor maintains a level at which the electrochemical reaction between/at the electrodes can be driven at an optimal/maximal rate so that an electric current can flow between the electrodes and can form the basis of the measurement of the analyte data and/or the oxygen data and/or other data.

Said possible resistor may be also part of the sensor circuit of the analyte sensor.

Furthermore, the electrodes can be operated periodically in an open circuit mode.

Then, the electrode(s) voltage(s) that can be measured can reflect the difference in the electrochemical potential of the first electrode / the working electrode and the second electrode / the counter electrode / combined counter / reference electrode.

While, for example, at the first electrode / the working electrode, analyte, e.g. glucose, is available in excess, but controlled / regulated / limited by the above exemplary described membrane, an oxygen reservoir at the second electrode / counter electrode can be depleted after short times, e.g. within a few minutes.

Hence, differences in the oxygen level can dominate the changes in the open circuit potential.

Hence, measuring the electric potential of the electrodes at the beginning and the end of a short open circuit phase, i.e. when the electrodes of the analyte sensor are operated in said open circuit mode, can reveal sufficient information to:

a) reconstruct the overall oxygen level at the analyte sensor site, and

b) from the recovery of the oxygen flux rate, the transport properties of the filling material or filling material layer or diffusion limiting layer can be measured, which in turn is proportional to the sensitivity of the analyte sensor.

For example, said sensitivity of the analyte sensor can be described by the differential slope of the current-glucose relation.

Further advantages of this operation scheme and of the design of the herein described analyte sensor can therefore comprise:

- Offering oxygen data (e.g. oxygen saturation / oxygen saturation level / oxygen saturation concentration in the body fluid) as auxiliary data to the patient or user, which can inter alia provide useful therapeutic information, e.g. being indicative of inflammations.

- Reduced power consumption of the analyte sensor.

- Reduced material wear at second electrode / counter electrode, thereby extending lifetime of the analyte sensor.

For completeness, it is noted that the analyte sensor can also be configured for harvesting the voltage / current or energy produced by electrochemical reactions at the electrodes of the analyte sensor and using the harvested energy for the operations of the analyte sensor.

Furthermore, the analyte sensor can be configured for being removably / detachably attached to a mounting unit for placing the analyte sensor on the skin of a user and for maintaining the analyte sensor in position.

The mounting unit may further comprise an inserting element, e.g. a cannula, configured for facilitating the guiding of the end portions of the electrodes of the analyte sensor during partial insertion of the electrodes into the skin / body of the user for taking analyte measurements and/or other measurements.

Said exemplary mounting unit can be implemented as a patch unit that can be configured to be removably attached to the skin / body of a user and that can be configured to receive and hold the analyte sensor such that the analyte sensor can perform analyte measurements of the user over a period of time while being coupled to the user via the mounting unit / patch unit.

However, it is also conceivable that the mounting unit or patch unit is integrated in the analyte sensor.

The analyte sensor may comprise a digital communication interface configured for transmitting, e.g. wireless, data measured by the analyte sensor to a data analysis unit, e.g. a data analysis unit of a continuous analyte, e.g. glucose, monitor system.

However, the analyte sensor itself may comprise one or more processors for analyzing data, e.g. glucose data and/or oxygen data and/or other data, measured by the analyte sensor.

The analyte sensor may also comprise means for outputting analyte data, e.g. glucose data, and/or other data, e.g. oxygen data (such as oxygen saturation / oxygen saturation level / oxygen saturation concentration in the body fluid, e.g. in blood or interstitial fluid), measured by the analyte sensor and/or data derived from the measured analyte data and/or further other data via visual and/or audible and/or tactile indications.

For example, the analyte sensor may comprise a display for displaying measured data, e.g. measured analyte data, e.g. glucose data, or oxygen data, and other information.

Exemplary material properties of the at least one impermeable membrane

In the following, further exemplary possible properties of the above and herein exemplary described at least one membrane of the analyte sensor that comprises a membrane material that is impermeable to the analyte / the analyte carrying fluid are described.

For example, said exemplary impermeable membrane material or nearly impermeable membrane material may refer to a membrane material that can be characterized by having a water uptake of less than 5% or less than 2% or less than 1% by weight, based on the total weight of the at least one membrane, as determined according to the Standard ASTM 570 of the American Society for Testing and Materials.

Stated differently, said exemplary membrane / membrane material can be hydrophobic.

Said exemplary membrane / membrane material can comprise a polymer, in particular a hydrophobic polymer, wherein, for example, said exemplary hydrophobic polymer can be characterized by having a water uptake of less than 5% or less than 2% or less than 1% by weight, based on the total weight of the hydrophobic polymer or membrane, as determined according the Standard ASTM 570 of the American Society for Testing and Materials.

Furthermore, said exemplary hydrophobic polymer can be a thermoplastic hydrophobic polymer.

For example, the hydrophobic polymer can have a glass transition temperature in the range from -100°C to 0°C, preferably in the range from -70°C to -50°C.

Herein, the glass transition temperature may be measured via differential scanning calorimetry using a ramp of 10°C/min for heating and cooling, wherein the glass transition temperature can be measured during the second heating cycle.

In other words, the hydrophobic polymer can first be heated with a ramp of 10°C/min, then can be cooled with a ramp of 10°C/min and then can be heated again with a ramp of 10°C/min to determine the glass transition temperature.

Said glass transition temperature / glass transition temperature range can be particularly advantageous as it can result in a sufficiently high stability of the membrane / membrane material. The membrane / membrane material can then act as a protective layer of the analyte sensor, i.e. for the electrodes, such that if, for example, the analyte sensor is bent, in particular during use, the protective layer will maintain its integrity and can protect underlying analyte sensor components, such as electrodes, sensor circuitry and other electronic components, from damage.

Furthermore, the hydrophobic polymer may for example be characterized by having a crystallization temperature in the range from 50 °C to 100 °C, for example in the range from 75 °C to 85 °C. The crystallization temperature can be measured via differential scanning calorimetry using the same parameters as for the glass transition temperature.

Furthermore, the possible exemplary hydrophobic polymer for / of the at least one membrane, may be selected from the group consisting of polyurethanes, polyureas, polyolefins, poly(meth)acrylates, polyesters, polyethers, polyamides, polyvinylchlorides, polyvinylbutyral-co-vinylalcohol-co-vinylacetate, slicones, epoxy resins and UV hardening resins.

Particularly preferred can be polyurethanes and/or UV (ultraviolet light) hardening resins. The at least one membrane may additionally to the at least one polymer comprise conductive particles, such as conductive carbon particles.

Thus an analyte sensor can be preferred wherein the at least one membrane comprises at least one polymer selected from the group consisting of polyurethanes, polyureas, polyolefins, poly(meth)acrylates, polyesters, polyethers, polyamides, polyvinylchlorides, polyvinylbutyral-co-vinylalcohol-co-vinylacetate and UV hardening resins. Particularly preferred can be polyurethanes and/or UV hardening resins.

Particularly suitable polyureas can be selected from the group consisting of hydrophobic polyureas.

Particularly suitable polyolefins can be selected from the group consisting of aliphatic polyolefins and aromatic polyolefins, in particular from the group consisting of polyethylene, polypropylene, polybutylene, polyisoprene, polybutadiene and polystyrene.

The term poly(meth)acrylates within the context of the present invention relates to polyacrylates and to polymethacrylates. Particularly suitable polymethacrylates are for example butylmethacrylates.

Particularly suitable polyesters can be selected from the group consisting of polyethyleneterephthalate (PET) and polycarbonates.

Particularly suitable polyethers can be selected from the group consisting of polytetrahydrofuran (PTHF) and polyacetal, which is also known as polyoxymethylene (POM).

Particularly suitable UV hardening resins can be selected from the group consisting of acrylated silicones, acrylated urethanes, acrylated epoxides and UV curing ester resins, such as acrylated polyesters.

Particularly suitable polyurethanes can be selected from the group consisting of hydrophobic polyurethanes, in particular hydrophobic thermoplastic polyurethanes (TPU).

The hydrophobic thermoplastic polyurethane may comprise hard segments and soft segments in various ratios. Suitable hard segments usually comprise a polymerization product of a diisocyanate and a polyol. A suitable diisocyanate may be an aliphatic diisocyanate or an aromatic diisocyanate, preferably an aliphatic diisocyanate.

Suitable aromatic diisocyanates are, for example, 4,4'-methylene diphenyl diisocyanate, and/or toluene-2,4-diisocyanate.

Suitable aliphatic diisocyanates are for example, hexamethylene diisocyanate, and/or isophorone diisocyanate.

A suitable polyol is preferably a diol, such as 1,4-butanediol, 1,5-pentanediol, 1,6-hexanediol, and/or 1,10-decanediol.

Suitable soft segments may comprise polyethers and/or polyesters. Suitable polyethers are, for example, polyethylene oxide and/or polytetrahydrofuran, whereas suitable polyesters are for example polyethylene terephthalate and/or polyethylene naphthalate.

Exemplary properties of filling material / filling material layer / diffusion limiting layer

In the following, further exemplary possible properties of the further above and herein exemplary discussed material / layer / filling material / filling layer / diffusion limiting layer that is permeable to the analyte carrying fluid / analyte and with which the at least opening of the at least one membrane can be filled, are described.

As previously mentioned, said filling material / filling layer / diffusion limiting layer may comprise an at least one polymeric material, e.g. a hydrophilic polymer.

Suitable polymeric materials may, for example be selected from the group consisting of a polyvinylpyridine based copolymer, a polyurethane and a hydrogel. Polyvinylpyridine based copolymers are particularly suitable.

Suitable hydrogels are in particular polyethylene glycol copolymers (PEG copolymers), polyvinyl acetate copolymers (PVA copolymers), poly(2-alkyl-2-oxazonline) copolymers, polyacrylate and/or methacrylate-acrylate copolymers or block-copolymers, in particular polyacrylate and/or methacrylate-acrylate copolymers or block-copolymers comprising hydrophilic side groups. Thus, as an example, suitable hydrogels may be selected from the group consisting of (hydroxyethyl)methacrylate (HEMA) -homopolymers, HEMA-copolymers, silicon hydrogels and HEMA-coN-vinylpyrrolidone-polymers, each of which may comprise side groups selected from the group consisting of methacrylic acid, glycerol methacrylate, N,N-dimethylacrylamide and phospharylcholine.

Specifically, the polymeric material may have a weight average molecular weight (MW) of more than 10.000 kDa. More specifically, the polymeric material may have a weight average molecular weight (MW) of more than 50.000 kDa, or even more than 100.000 kDa. Particularly suitable are polymeric materials with a weight average molecular weight (MW) of 10.000 to 500.000 kDa.

The above and herein exemplary described analyte sensor may be part of a continuous analyte monitoring system, e.g. a continuous glucose monitoring (CGM) system, comprising: a medical device comprising an analyte sensor as exemplary described herein and one or more processors configured for processing data collected by the analyte sensor.

Furthermore, the above and herein exemplary described analyte sensor may be part of a kit comprising: at least one analyte sensor as described above, and at least one mounting unit, e.g. a patch unit, configured for the placement of the at least one analyte sensor on the skin of a user and to which the at least one analyte sensor can be detachably coupled.

The following figures illustrate exemplary the following aspects of the invention.

**Fig.1a**: Exemplary structure / exemplary structural aspects of the first electrode / the working electrode of an exemplary analyte sensor

**Fig.1b:** Exemplary alternative structure / exemplary alternative structural aspects of the first electrode / the working electrode of an exemplary analyte sensor

**Fig. 1c:** Exemplary further alternative structure / exemplary further alternative structural aspects of the first electrode / working electrode of an exemplary analyte sensor

**Fig. 1d:** Exemplary structure / exemplary structural aspects of the second electrode / the counter electrode of an exemplary analyte sensor

**Fig.1e:** Exemplary cross section of an exemplary analyte sensor

**Fig. 2:** Exemplary flow diagram of an exemplary method for assembling / manufacturing an exemplary analyte sensor

**Fig. 3:** Exemplary flow diagram of an exemplary method for the operation of an exemplary analyte sensor

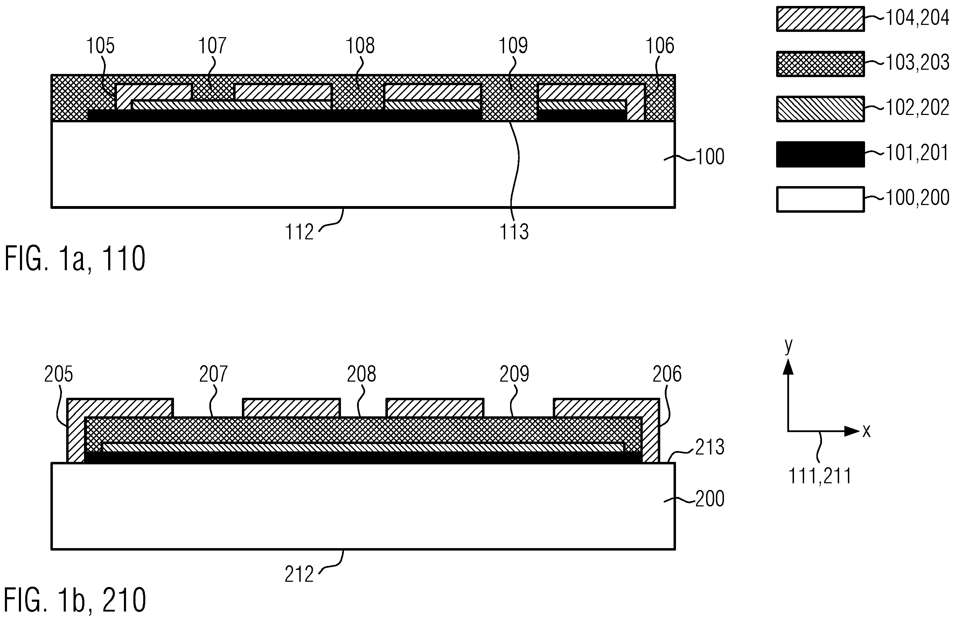

**Fig. 1a** shows an exemplary cross section of a part of an exemplary analyte sensor, wherein a possible multi-layer structure 110 of the first electrode / the working electrode 101 of the analyte sensor is illustrated.

The second electrode / the counter electrode is not shown.

The analyte sensor may comprise a substrate 100, as described further above, which can carry the various layers and components of the analyte sensor.

For example, the first electrode / the working electrode 101 and/with the sensor circuit can be mounted directly onto the top surface 113 of the substrate 100.

The (not shown) second electrode / the counter electrode and its various layers and components could then for example be mounted to the bottom surface 112 of the substrate 100.

The first electrode / the working electrode 101 can be covered by / can comprise a sensing layer 102 / sensing material, as described further above.

Furthermore, the first electrode / the working electrode 101 can be covered by at least one membrane 104 and which can be applied / be mounted / be arranged above the sensing layer 102, as described further above. As also described above, the membrane 104 can comprise a membrane material that is impermeable to the analyte or the analyte carrying fluid.

The at least one membrane 104 can comprise at least one opening. In the present case, three exemplary openings 107, 108, 109 are shown / visible and through which the analyte or the analyte carrying fluid can pass through.

The at least opening can be filled with a material 103 that is permeable to the analyte / analyte carrying fluid, e.g. a hydrophilic polymer. In the example shown, all openings 107, 108, 109 are fully filled with the material 103 that is permeable to the analyte / analyte carrying fluid.

In the example shown, the filling material 103 that is permeable to the analyte carrying fluid also covers the at least one membrane 104, i.e. the filling material 103 / filling layer is embedding the at least one membrane 104. The filling material layer can also be referred to as diffusion limiting layer, as it can control the diffusion of the analyte / analyte carrying fluid through the openings 107, 108, 109.

As previously described, the at least one opening / the openings of the membrane can be of different types / different depths. In case of a plurality of openings, all openings can be of the same type or of different types, or can be grouped in groups of different types.

In the shown example, three exemplary possible different opening types are illustrated.

For example, opening 107 illustrates an exemplary first type, wherein the opening is formed such, that it penetrates / extends only through the membrane 104 and not to any possible further layers of the analyte sensor below the membrane 104.

For example, opening 108 illustrates an exemplary second type, wherein the opening is formed such, that it penetrates / extends through the at least one membrane and at least partially or fully further extends to / extends through the first layer below the membrane, e.g., as shown, the sensing layer 102.

For example, opening 109 illustrates an exemplary third type, wherein the opening may be formed such, that it penetrates / extends through the at least one membrane and further extends through any other layer below the membrane including extending partially of fully through an/the electrode lying below the membrane, e.g. in the example shown, the opening 109 extends penetrates / extends through the membrane 104, the sensing layer 102 and the first electrode / the working electrode 101.

As also previously described, the at least one membrane can be applied / configured such, that its possible edges / borders / lateral sides can wrap at least partially or fully around the edges / borders / lateral sides of the layers and/or components lying below the at least one membrane.

Here, two exemplary possible edge types are illustrated.

For example, the shown edge 105 of the at least one membrane is of a first type, wherein the edge is configured such, as to wrap in an L-shaped manner, e.g. with the long side of the L being parallel to the substrate 100 and with the short side of the L being orthogonal to the substrate 100, around the first layer 102 below the membrane, e.g. around the sensing layer 102.

The shown edge 106 of the at least one membrane is of a second type, wherein the edge is configured such, as to wrap in said L-shaped manner around all layers / all components below the membrane, e.g. around the sensing layer 102 and around the electrode, e.g. around the first electrode / the working electrode, until making contact with / touching the substrate 100.

The edge types of all possible different edges of the at least one membrane can be of the same or of different type. The shown configuration is just exemplary.

Exemplary orthogonal reference system 111 indicates the orientation of the **Fig.1a** and its components, wherein the top surface 113 and the bottom surface 112 of the substrate 100 are parallel to each other and parallel to the horizontal x-axis, and wherein, for example, the orthogonal y-axis is aligned or parallel to the direction of Earth's gravity.

Fig. 1b shows an exemplary cross section of a part of an exemplary alternative analyte sensor, wherein an alternative possible multi-layer structure 210 of the first electrode / the working electrode 201 of the analyte sensor is illustrated.

The second electrode / the counter electrode is not shown.

The analyte sensor may comprise a substrate 200, as described further above, which can carry the various layers and components of the analyte sensor.

For example, the first electrode / the working electrode 201 and/with the sensor circuit can be mounted directly onto the top surface 213 of the substrate 200.

The (not shown) second electrode / the counter electrode and its various layers and components could then for example be mounted to the bottom surface 212 of the substrate 200.

The first electrode / the working electrode 201 can be covered by / can comprise a sensing layer 202 / sensing material, as described further above.

Furthermore, the sensing layer 202 / sensing material can be covered by at least one layer of filling material 203 that is permeable to the analyte carrying fluid.

The at least one membrane 204 can be applied / be mounted / be arranged above the sensing layer 202 and can encase / embed the filling material / filling material layer 203 and the sensing layer 202.

As described above, the membrane 204 can comprise a membrane material that is impermeable to the analyte or the analyte carrying fluid and the membrane 204 can comprise openings 207, 208, 209 through which the analyte or the analyte carrying fluid can pass through.

The exemplary edges of the at least one membrane 204 are exemplary shown as being of a second type, wherein the edges are configured such, as to wrap in said L-shaped manner around all layers / all components below the membrane, e.g. around the filling material layer 203, around the sensing layer 202 and around the electrode, e.g. around first electrode / the working electrode, until making contact with / touching the substrate 200.

In the shown example, three exemplary opening 207, 208, 208 of the at least one membrane 204 are shown / are visible, wherein said openings are all of the same exemplary type.

More specifically, the openings are all of said exemplary first type, wherein the opening is formed such, that it penetrates / extends only through the membrane 204 and not to any possible further layers of the analyte sensor below the membrane 204, e.g. as shown here, the openings do not penetrate/ do not extend through the filling material layer 203, not through the sensing layer 202 and not through the first electrode / the working electrode 201.

However, it is also conceivable that the openings are penetrating through all layers 203, 202, 201 and components carried by the substrate 200 and may even extend partially into the substrate.

Exemplary orthogonal reference system 211 is identical to orthogonal reference system 111 and indicates the orientation of the Fig.1b and its components, wherein the top surface 213 and the bottom surface 212 of the substrate 200 are parallel to each other and parallel to the horizontal x-axis, and wherein, for example, the orthogonal y-axis is aligned or parallel to the direction of Earth's gravity.

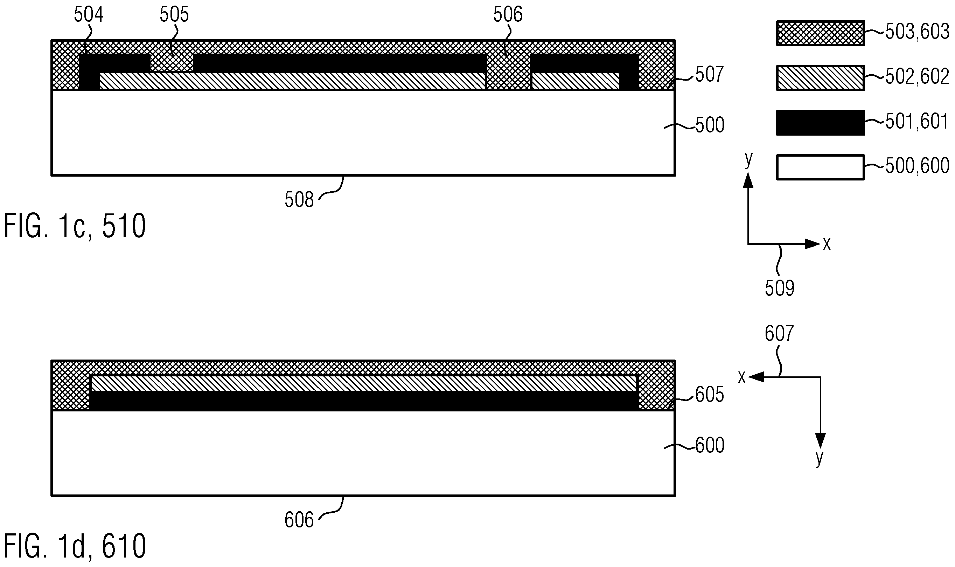

**Fig. 1c** shows an exemplary cross section of a part of an exemplary further alternative analyte sensor, wherein another alternative possible multi-layer structure 510 of the first electrode / the working electrode 501 of the analyte sensor is illustrated.

The second electrode / the counter electrode is not shown.

The analyte sensor may comprise a substrate 500, as described further above, which can carry the various layers and components of the analyte sensor.

For example, the first electrode / the working electrode 501 and/with the sensor circuit can be mounted directly onto the top surface 507 of the substrate 500.

The (not shown) second electrode / the counter electrode and its various layers and components could then for example be mounted to the bottom surface 508 of the substrate 500.

The first electrode / the working electrode 501 can comprise a sensing layer 502 / sensing material, as described further above, which in contrast to the multi-layer structures of **Fig.1a** and **Fig.1b** can be in direct contact with the substrate 500, i.e. with the top surface 507 of the substrate 500.

In other words, the sensing layer 502 / sensing material can be arranged below the first electrode / the working electrode 501.

Instead of having a dedicated membrane with membrane material that is impermeable to the analyte or the analyte carrying fluid and with openings through which the analyte or the analyte carrying fluid can pass through, as is the case for the embodiments of Fig.1a and Fig. 1b, it is the first electrode / the working electrode 501 itself, which acts as an analyte flux localization component.

In other words, the working electrode 501 can comprise a material, e.g. carbon, that is impermeable to the analyte or the analyte carrying fluid but the working electrode can also comprise openings 505, 506 through which the analyte or the analyte carrying fluid can pass through to come into contact with an electrochemical active layer, e.g. sensing layer 502.

The openings of the working electrode 501 can be of the same or similar types as described above. In the shown example, opening 505 penetrates through the working electrode 501 but not through the sensing layer 502 and opening 506 penetrates through the working electrode 501 and through the sensing layer 502, but not into the substrate 500.

Also the edges of the working electrode 501 can be configured according to various types. In the shown example, the edge 504 wraps around the edge or lateral side of the sensing layer 502.

The openings 505, 506 of the working electrode 501 can be filled with a material 503 that is permeable to the analyte carrying fluid, e.g. a hydrophilic polymer. In the example shown, all openings 505, 506 are fully filled with the material 503 that is permeable to the analyte carrying fluid.

In the example shown the filling material 503 that is permeable to the analyte carrying fluid also covers the working electrode, i.e. the filling material 503 / filling layer is embedding the working electrode.

Exemplary orthogonal reference system 509 indicates the orientation of the Fig.1c and its components, wherein the top surface 507 and the bottom surface 508 of the substrate 500 are parallel to each other and parallel to the horizontal x-axis, and wherein, for example, the orthogonal y-axis is aligned or parallel to the direction of Earth's gravity.

**Fig.1d** shows an exemplary cross section of a part of an exemplary analyte sensor, wherein a possible multi-layer structure 610 of the second electrode / the counter electrode 601 of the analyte sensor is illustrated.

The first electrode / the working electrode is not shown

The analyte sensor may comprise a substrate 600, as described further above, which can carry the various layers and components of the analyte sensor.

In contrast to the orientations of **Fig.1a**, **Fig.1b** and **Fig.1c**, the orientation of **Fig. 1d** is such that the substrate has been rotated by 180° as indicated by orthogonal reference system 607, i.e. the shown upper surface of the substrate is actually the bottom surface 605 of the substrate in the orthogonal reference systems 111, 211, 509.

For example, the second electrode / the counter electrode 601 and the sensor circuit components can be mounted directly onto the bottom surface 605 of the substrate 600.

The (not shown) first electrode / the working electrode and its various layers and components could then for example be mounted to the top surface 606 of the substrate 600.

The second electrode / the counter electrode 601 can be covered by a sensing material layer 602.

Furthermore, a layer of filling material / a diffusion limiting layer 603 that is permeable to the analyte and/or analyte carrying fluid can cover the sensing material layer 602.

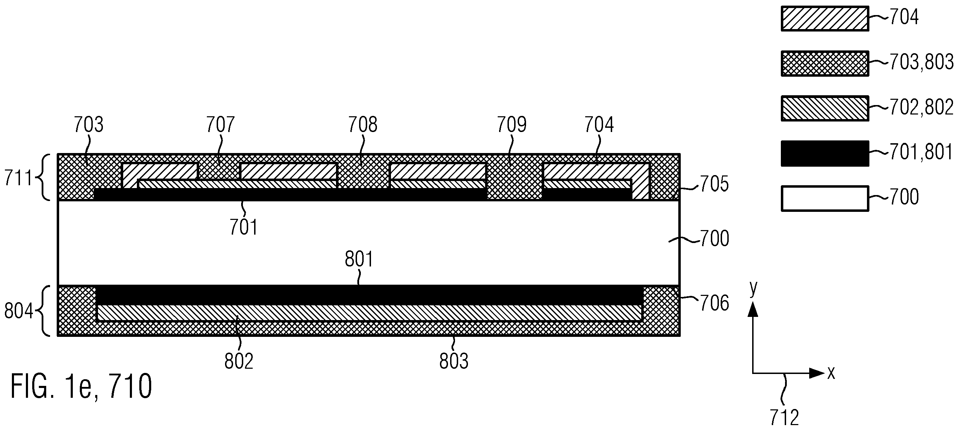

**Fig.1e** shows an exemplary cross section of a part of an exemplary analyte sensor, wherein both the first electrode / the working electrode 701 and the second electrode / the counter electrode 801 attached to opposing sides of substrate 700 are visible.

In this example, the multi-layer structure 711 of the working electrode 701 is mounted onto the top surface 705 of the substrate 700 and corresponds to the multi-layer structure 110 of the working electrode 101 described in **Fig.1a**.

Hence, the working electrode 701 can be covered by / can comprise a sensing layer 702 / sensing material, as described further above.

The sensing layer 702 can be covered by at least one membrane 704.

Said membrane 704 can comprise a membrane material that is impermeable to the analyte or the analyte carrying fluid and can comprise openings 707, 708, 709 through which the analyte or the analyte carrying fluid can pass through. Said opening are of exemplary different types as described in **Fig. 1a**.

In the shown example, the openings 707, 708, 709 are fully filled with a filling material 703 that is permeable to the analyte / analyte carrying fluid.

In the example shown, the filling material 703 that is permeable to the analyte carrying fluid also covers the at least one membrane 704, i.e. the filling material 103 or filling material layer is embedding the at least one membrane 704. The filling material layer can also be referred to as diffusion limiting layer, as it can control the diffusion of the analyte / analyte carrying fluid through the openings 707, 708, 709.

In this example, the multi-layer structure 804 of the counter electrode 801 is mounted onto the bottom surface 706 of the substrate 700 and corresponds to the multi-layer structure 610 of the counter electrode 601 described in **Fig.1d**.

The counter electrode 801 can be covered by a sensing material layer 802.

Furthermore, a layer of filling material / a diffusion limiting layer 803 that is permeable to the analyte and/or analyte carrying fluid can cover the sensing material layer 802.

The here shown combination and arrangement of the multi-layer structures of the working electrode and the counter electrode is exemplary only. Other combinations are conceivable. For example a combination of the embodiments according to **Fig.1b** or **Fig.1c** with the embodiment of **Fig.1d**.

While in the shown example, the working electrode 701 and the counter electrode 801 are arranged directly opposed to each other on different sides of the substrate, they can be also arranged with an offset, e.g. a horizontal offset along the x-axis, to each other.

Also a coplanar arrangement of the working electrode 701 and the counter electrode 801 on the same side, e.g. the top surface, of the substrate is conceivable.

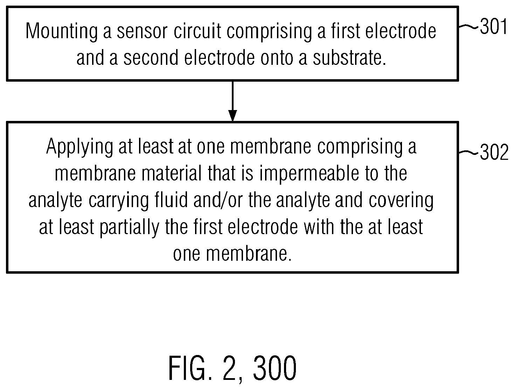

**Fig. 2** shows an exemplary flow diagram for an exemplary method 300 for assembling / manufacturing an exemplary analyte sensor of the kind exemplary described above and that can be used in medical devices or medical continuous monitoring devices for measuring analyte data in an analyte carrying fluid of a patient, in particular for measuring glucose data and/or oxygen data and/or other data.

Said exemplary method may comprise a step of mounting 301 a sensor circuit comprising a first electrode, i.e. a working electrode, and a second electrode, i.e. a counter electrode, onto a substrate. The first electrode and the second electrode can be arranged on opposing sides or opposing surfaces of the substrate or on the same side or same surface of the substrate.

The method can further comprise a step of applying 302 at least at one membrane comprising a membrane material that is impermeable to the analyte and/or impermeable to the analyte carrying fluid and covering fully or at least partially the first electrode with the at least one membrane. Optionally also the second electrode / the counter electrode can be fully or at least partially covered with the at least one membrane.

The method may inter alia further comprise forming at least one opening in the membrane, for example by a drilling and/or ablation process, e.g. by laser ablation.

The one or more openings in the membrane are formed in the part of the membrane that fully or at least partially covers the first electrode / the counter electrode.

Additionally, the openings in the membrane can be filled at least partially with a filling material that is permeable to the analyte carrying fluid and/or the analyte, e.g. a hydrophilic polymer.

Furthermore, the filling material can be applied such as to form a filling material layer that covers and/or embeds the membrane. Said filling material layer can also be referred to as diffusion limiting layer.

In addition the filling material layer may also be applied to cover the second electrode / the counter electrode.

As previously described the electrodes, e.g. the first electrode / the working electrode and/or the second electrode / the counter electrode, can be configured with or can be equipped with an electrochemically active layer, i.e. with a sensing material layer as described further above.

The exemplary steps of applying a layer or a membrane to the analyte sensor and its components may comprise dip coating steps.

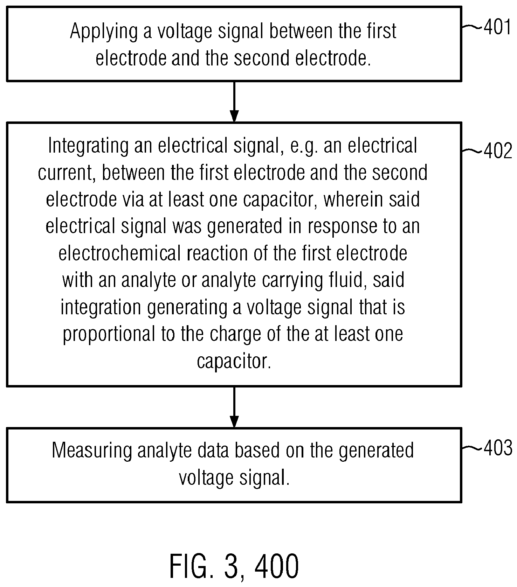

Fig. 3 shows an exemplary flow diagram of an exemplary method 400 for the operation of an exemplary analyte sensor of the kind exemplary described above may comprise a step of applying 401 a voltage signal between the first electrode and the second electrode.

The method may further comprise a step of integrating 402 an electrical signal, e.g. an electrical current, between first electrode and the second electrode via at least one capacitor, wherein said electrical signal was generated in response to an electrochemical reaction of the first electrode with an analyte or analyte carrying fluid, and wherein said integration generates a voltage signal that is proportional to the charge of the at least one capacitor.

Said generated voltage signal can then serve as basis for measuring 403 analyte data and/or oxygen data and/or other data with the analyte sensor.

Furthermore, the method may comprise operating the electrodes periodically in an open circuit mode.

Followed by Fig. 1a, Fig.1b, Fig. 1c, Fig. 1d, Fig. 1e, Fig. 2 and Fig. 3, wherein the reference signs denote the following exemplary aspects, exemplary characteristics and exemplary steps.

**100** Exemplary substrate
**101** Exemplary first electrode / working electrode
**102** Exemplary sensing layer / sensing material / electrochemical active layer
**103** Exemplary filling material / filling material layer of material that is permeable to the analyte / analyte carrying fluid, exemplary diffusion limiting layer
**104** Exemplary membrane / membrane layer / membrane material that is impermeable to the analyte / analyte carrying fluid, exemplary flux localization membrane / flux localization layer
**105** Exemplary edge of membrane, exemplary edge of first type
**106** Exemplary edge of membrane, exemplary edge of second type
**107** Exemplary opening in membrane, exemplary opening of first type
**108** Exemplary opening in membrane, exemplary opening of second type
**109** Exemplary opening in membrane, exemplary opening of third type
**110** Exemplary structure, exemplary multi-layer structure of analyte sensor / first electrode / working electrode
**111** Exemplary orthogonal reference system
**112** Exemplary bottom surface of substrate
**113** Exemplary top surface of substrate
**200** Exemplary substrate
**201** Exemplary first electrode / working electrode
**202** Exemplary sensing layer / sensing material
**203** Exemplary filling material / filling material layer of material that is permeable to the analyte / analyte carrying fluid, exemplary diffusion limiting layer

**204** Exemplary membrane / membrane layer / membrane material that is impermeable to the analyte / analyte carrying fluid, exemplary flux localization membrane / flux localization layer
**205** Exemplary edge of membrane, exemplary edge of second type
**206** Exemplary edge of membrane, exemplary edge of second type
**207** Exemplary opening in membrane, exemplary opening of first type
**208** Exemplary opening in membrane, exemplary opening of first type
**209** Exemplary opening in membrane, exemplary opening of first type
**210** Exemplary structure, exemplary multi-layer structure of analyte sensor / first electrode / working electrode
**211** Exemplary orthogonal reference system
**212** Exemplary bottom surface of substrate
**213** Exemplary top surface of substrate
**300** Exemplary method for assembling / manufacturing an exemplary analyte sensor
**301**, **302** Exemplary method steps
**400** Exemplary method for operating an exemplary analyte sensor
**401**, **402**, **403** Exemplary method steps
**500** Exemplary substrate
**501** Exemplary first electrode / working electrode
**502** Exemplary sensing layer / sensing material / electrochemical active layer
**503** Exemplary filling material / filling material layer of material that is permeable to the analyte / analyte carrying fluid, exemplary diffusion limiting layer
**504** Exemplary edge of working electrode
**505** Exemplary opening
**506** Exemplary opening
**507** Exemplary top surface of substrate
**508** Exemplary bottom surface of substrate
**509** Exemplary orthogonal reference system
**600** Exemplary substrate
**601** Exemplary second electrode / counter electrode
**602** Exemplary sensing layer / sensing material / electrochemical active layer
**603** Exemplary filling material / filling material layer of material that is permeable to the analyte / analyte carrying fluid, exemplary diffusion limiting layer
**605** Exemplary bottom surface of substrate
**606** Exemplary top surface of substrate
**607** Exemplary orthogonal reference system
**700** Exemplary substrate
**701** Exemplary first electrode / working electrode
**702** Exemplary sensing layer / sensing material / electrochemical active layer
**703** Exemplary filling material / filling material layer of material that is permeable to the analyte / analyte carrying fluid, exemplary diffusion limiting layer
**704** Exemplary membrane / membrane layer / membrane material that is impermeable to the analyte / analyte carrying fluid, exemplary flux localization membrane / flux localization layer
**705** Exemplary top surface of the substrate
**706** Exemplary bottom surface of the substrate
**707** Exemplary opening
**708** Exemplary opening
**709** Exemplary opening
**710** Exemplary structure, exemplary multi-layer structure of analyte sensor
**711** Exemplary structure, exemplary multi-layer structure of working electrode of analyte sensor
**712** Exemplary orthogonal reference system
**801** Exemplary second electrode / counter electrode
**802** Exemplary sensing layer / sensing material / electrochemical active layer
**803** Exemplary filling material / filling material layer of material that is permeable to the analyte / analyte carrying fluid, exemplary diffusion limiting layer
**804** Exemplary structure, exemplary multi-layer structure of counter electrode of analyte sensor

**Claims**

1. Analyte sensor for use in medical devices for measuring analyte data in an analyte carrying fluid, in particular for measuring glucose data, comprising:

   a first electrode (101) being a working electrode,
   a second electrode,
   a substrate (100) carrying the first electrode and the second electrode,
   at least one membrane (104) which at least partially covers the first electrode (101),
   wherein the at least one membrane (101) comprises a membrane material that is impermeable to the analyte carrying fluid and/or the analyte,
   wherein the at least one membrane comprises at least one opening (107),
   and wherein the at least one membrane (104) is configured for controlling the flux of the analyte carrying fluid and/or the analyte to the first electrode (101) via the at least one opening (107).

2. Analyte sensor according to the preceding claim, wherein the diameter of the at least one opening (107) is similar to the thickness of the at least one membrane (104) , e.g. in the range of 10 to 100 micrometers.

3. Analyte sensor according to one of the preceding claims, wherein the at least one opening (107) has been formed by a laser ablation process.

4. Analyte sensor according to one of the preceding claims, wherein the at least one opening (107) is at least partially filled with a material (103) that is permeable to the analyte carrying fluid and/or the analyte, e.g. a hydrophilic polymer that is optionally selected from the group consisting of a polyvinylpyridine based copolymer, a polyurethane and a hydrogel.

5. Analyte sensor according to the preceding claim, wherein the material (103) that is permeable to the analyte carrying fluid and/or the analyte is covering at least partially the first electrode (101) and/or the second electrode.

6. Analyte sensor according to one of the preceding claims, wherein the membrane (104) is hydrophobic.

7. Analyte sensor according to one of the preceding claims, further comprising a sensing layer (102), wherein the sensing layer (102) is configured for providing at least one enzyme to the first electrode (101).

8. Analyte sensor according to one of the preceding claims, wherein the first electrode (101) and the second electrode are arranged on opposing sides of the substrate (100).

9. Analyte sensor according to one of the preceding claims, wherein the second electrode is an electrode selected from a group comprising the following types: a counter electrode and a combined counter/reference electrode and/or wherein the second electrode is an oxygen electrode that is configured to use oxygen solved in the analyte carrying fluid as electron acceptor and wherein the second electrode can comprise gold or platinum as conductive material.

10. Method (300) for manufacturing an analyte sensor according to any one of the previous claims for use in medical devices for measuring analyte data in an analyte carrying fluid, in particular for measuring glucose data, comprising:

    mounting (301) a sensor circuit comprising a first electrode and a second electrode onto a substrate, the first electrode being a working electrode,
    applying (302) at least at one membrane comprising a membrane material that is impermeable to the analyte carrying fluid and/or the analyte and
    covering at least partially the first electrode with the at least one membrane,
    forming at least one opening in the membrane, for example by a drilling and/or ablation process, e.g. by laser ablation, and optionally
    filling, at least partially the least one opening in the membrane with a material (103) that is permeable to the analyte carrying fluid and/or permeable to the analyte.

11. Analyte sensor for use in medical devices for measuring analyte data in an analyte carrying fluid, in particular for measuring glucose data, comprising:

    a first electrode (101) being a working electrode, and
    a second electrode,
    a substrate (100) carrying the first electrode and the second electrode,
    wherein the first electrode comprises a material that is impermeable to the analyte carrying fluid and/or impermeable to the analyte, and wherein first electrode comprises at least one opening, and wherein the first electrode is configured for controlling the flux of the analyte carrying fluid and/or the analyte to an electrochemical active layer of the first electrode via the at least one opening.

12. Method (400) for operating an analyte sensor according to one of the preceding analyte sensor claims 1 to 11, comprising:

    applying (401) a voltage signal between the first electrode and the second electrode,
    integrating (402) an electrical signal, e.g. an electrical current, between the first electrode and the second electrode via at least one capacitor, wherein said electrical signal was generated in response to an electrochemical reaction of the first electrode with an analyte or analyte carrying fluid, said integration generating a voltage signal that is proportional to the charge of the at least one capacitor,
    measuring (403) analyte data based on the generated voltage signal.

13. Method (400) according to the preceding claim, wherein the electrodes are operated periodically in an open circuit mode.

14. Continuous analyte monitoring system, e.g. a continuous glucose monitoring system, comprising:
    a medical device comprising an analyte sensor according to one of the preceding analyte sensor claims, and one or more processors configured for processing data collected by the analyte sensor.

15. Kit comprising:

    at least one analyte sensor according to one of the preceding analyte sensor claims,
    at least one mounting unit, e.g. a patch unit, configured for the placement of the at least one analyte sensor on the skin of a user and to which the at least one analyte sensor can be detachably coupled.

FIG. 1a, 110

FIG. 1b, 210

EP 4 278 966 A1

504    505                                              506

507

500

508

**FIG. 1c, 510**

503,603

502,602

501,601

500,600

y

x

509

607

x

605

600

y

606

**FIG. 1d, 610**

EP 4 278 966 A1

FIG. 1e, 710

EP 4 278 966 A1

Mounting a sensor circuit comprising a first electrode and a second electrode onto a substrate. ~301

Applying at least at one membrane comprising a membrane material that is impermeable to the analyte carrying fluid and/or the analyte and covering at least partially the first electrode with the at least one membrane. ~302

FIG. 2, 300

Applying a voltage signal between the first electrode and the second electrode. ~401

Integrating an electrical signal, e.g. an electrical current, between the first electrode and the second electrode via at least one capacitor, wherein said electrical signal was generated in response to an electrochemical reaction of the first electrode with an analyte or analyte carrying fluid, said integration generating a voltage signal that is proportional to the charge of the at least one capacitor. ~402

Measuring analyte data based on the generated voltage signal. ~403

FIG. 3, 400

Europäisches
Patentamt

European
Patent Office

Office européen
des brevets

# EUROPEAN SEARCH REPORT

**Application Number**

EP 22 17 3589

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/328877 A1 (VADDIRAJU SANTHISAGAR [US] ET AL) 15 November 2018 (2018-11-15) * abstract; figures 1-6 * * paragraphs [0002], [0017] – [0022], [0031] – [0081] * | 1-15 | INV. A61B5/145 A61B5/1473 A61B5/1486 A61B5/00 |
| X | US 2015/038410 A1 (FELDMAN BENJAMIN J [US] ET AL) 5 February 2015 (2015-02-05) * abstract; figures 1-7 * * paragraphs [0031] – [0087] * | 1-15 | |
| X | US 2016/235346 A1 (LIU ZENGHE [US] ET AL) 18 August 2016 (2016-08-18) * abstract; figures 1-9 * * paragraphs [0031] – [0109] * | 1-15 | |
| X | EP 0 539 625 A1 (DRAEGER MED ELECTRONICS BV [NL]) 5 May 1993 (1993-05-05) * abstract; claim 1; figures 1-3 * * column 1, lines 1-20 * * column 3, line 34 – column 6, line 16 * | 1-15 | TECHNICAL FIELDS SEARCHED (IPC) A61B |

The present search report has been drawn up for all claims

| Place of search | Date of completion of the search | Examiner |
|---|---|---|
| The Hague | 21 October 2022 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 3589

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2022

| Patent document cited in search report | Publication date | Patent family member(s) | Publication date |
|---|---|---|---|
| US 2018328877 A1 | 15-11-2018 | CA 3062760 A1 | 15-11-2018 |
| | | CN 110678122 A | 10-01-2020 |
| | | EP 3621521 A1 | 18-03-2020 |
| | | US 2018325430 A1 | 15-11-2018 |
| | | US 2018328877 A1 | 15-11-2018 |
| | | WO 2018208357 A1 | 15-11-2018 |
| US 2015038410 A1 | 05-02-2015 | AU 2008230130 A1 | 02-10-2008 |
| | | BR PI0808001 A2 | 17-06-2014 |
| | | CA 2676241 A1 | 02-10-2008 |
| | | CN 101686809 A | 31-03-2010 |
| | | EP 2111159 A1 | 28-10-2009 |
| | | JP 5324473 B2 | 23-10-2013 |
| | | JP 2010517054 A | 20-05-2010 |
| | | NZ 578344 A | 29-07-2011 |
| | | RU 2009132504 A | 10-03-2011 |
| | | TW 200934445 A | 16-08-2009 |
| | | US 2008179187 A1 | 31-07-2008 |
| | | US 2015038410 A1 | 05-02-2015 |
| | | US 2015323487 A1 | 12-11-2015 |
| | | US 2017114384 A1 | 27-04-2017 |
| | | WO 2008118257 A1 | 02-10-2008 |
| US 2016235346 A1 | 18-08-2016 | AU 2016220297 A1 | 27-07-2017 |
| | | CA 2976767 A1 | 25-08-2016 |
| | | CN 107249459 A | 13-10-2017 |
| | | EP 3258848 A1 | 27-12-2017 |
| | | JP 2018510672 A | 19-04-2018 |
| | | KR 20170117183 A | 20-10-2017 |
| | | US 2016235346 A1 | 18-08-2016 |
| | | WO 2016133841 A1 | 25-08-2016 |
| EP 0539625 A1 | 05-05-1993 | NONE | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0136660 A2

**Non-patent literature cited in the description**

- **FELDMAN et al.** *Diabetes Technology & Therapeutics,* 2003, vol. 5 (5), 769-779